# FASCICULE DE BREVET EUROPEEN

(11) **EP 0 869 814 B1**
(45) Date de publication et mention de la délivrance du brevet: **20.07.2005**
(21) Numéro de dépôt: 96943170.9
(22) Date de dépôt: 20.12.1996
(51) Int. Cl.: A61K 39/12, A61K 47/00

(54) **STABILISANTS POUR VACCINS VIVANTS**
STABILISATOREN FÜR LEBENDIMPFSTOFFE
STABILISERS FOR LIVE VACCINES

(30) Priorité: 22.12.1995 FR 9515778
(43) Date de publication de la demande: 14.10.1998
(73) Titulaire: Sanofi Pasteur, 69367 Lyon Cedex 07 (FR)
(72) Inventeur: FANGET, Bernard, F-69210 Saint-Germain-sur-l'Arbresle (FR); FRANCON, Alain, F-69690 Bessenay (FR)
(74) Mandataire: Ayroles, Marie-Pauline
(86) Numéro de dépôt international: PCT/FR1996/002054
(87) Numéro de publication internationale: WO 1997/023238

(56) Documents cités:
- EP-A- 0 065 905
- EP-A- 0 252 059
- EP-A- 0 353 108
- DD-A- 299 213
- DATABASE WPI Week 8208 Derwent Publications Ltd., London, GB; AN 82-14620E XP002012971 & JP 57 007 423 A (TAKEDA CHEMICAL IND KK) , 14 Janvier 1982

## Description

La présente invention concerne un procédé de préparation d'un vaccin contenant un virus de la varicelle vivant atténué.

Les vaccins comprenant des virus vivants et plus particulièrement des virus vivants atténués présentent une fragilité importante dans leurs conditions de préparation et de stockage. On constate d'importantes chutes du titre viral pendant les étapes de lyophilisation, durant le stockage et aussi pendant les étapes de récolte du virus après culture. Étant donné l'importance du titre viral dans l'efficacité de l'immunisation, le recours à des agents stabilisants destinés à préserver au mieux le titre viral s'avère indispensable. Les agents stabilisants sont des composés chimiques et/ou biologiques que l'on peut ajouter aux vaccins aux différents stades de leur préparation en vue de maintenir un niveau d'efficacité vaccinale lors de son utilisation qui peut parfois avoir lieu plusieurs années après le début du stockage.

De nombreux composés ont été testés pour leurs propriétés stabilisantes de différents vaccins contenant des virus vivants atténués. Les composés testés dans l'art antérieur (cf. par exemple US3,783,098, US 3,915,794, EP 028,563), dont l'un des premiers fut le SPGA contiennent essentiellement de la sérum-albumine bovine ou humaine, de la caséine plus ou moins hydrolysée ou encore de la gélatine, éventuellement un sel de métal alcalin de l'acide glutamique, un sucre (glucose, saccharose, dextran); et un phosphate monométallique ou dimétallique de métal alcalin ou un de leur mélanges.

EP 353 108 décrit un procédé de préparation d'un vaccin lyophilisé à virus atténué. Les virus bénéficiant de ce procédé sont ceux qui rencontrent des difficultés de conservation à l'état lyophilisé. Sont nommément cités les virus de la rougeole, de la rubéole et des oreillons. Les virus sont cultivés, récoltés et mélangés à un agent stabilisant, de préférence avant lyophilisation. En pratique, les virus sont récoltés et congelés ; puis décongelés et mélangés extemporanément à un agent stabilisant juste avant lyophilisation. L'urée est un composant essentiel de l'agent stabilisant. Dans les exemples, d'autres composants sont cités ; et notamment, lactose, sorbitol, mannitol, dextran et albumine humaine. Le saccharose n'est pas répertorié.

EP 065 905 décrit un composé stabilisant pour le vaccin contre la fièvre jaune qui comporte un ou plusieurs acides aminés choisis dans un groupe de onze acides aminés, du lactose, du sorbitol et une solution tampon phosphate (PBS).

JP 57 007 423 décrit des compositions à base de disaccharide et de polyalcool dans un tampon phosphate. Le disaccharide peut être de manière indifférenciée, du saccharose ou du lactose. Ces compositions sont utilisées après la récolte du virus de la rougeole, pour stabiliser ce dernier lors de sa congélation.

DD-A-299 213 mentionne qu'il peut être fait usage de saccharose dans la composition de l'agent stabilisant qui est utilisé pour stabiliser le virus de la rougeole uniquement au cours de sa lyophilisation. La récolte n'est pas faite en présence d 'agent stabilisant. En effet, le virus de la rougeole est un virus qui sort de lui-même des cellules. Pour le récolter, il suffit simplement de centrifuger la culture et de récupérer le surnageant. Il n'y a pas besoin de lyser les cellules pour récolter ce virus. L'agent stabilisant est ajouté dans le surnageant ; donc après récolte.

Les compositions de l'art antérieur présentent généralement des inconvénients majeurs. En premier lieu, elles contiennent des protéines ou des hydrolysats de protéines d'origines animales ou humaines (albumines...), qui représentent un risque biologique potentiel et ne constituent pas des composants chimiquement définis. Par ailleurs, les compositions de l'état de la technique ne confèrent pas une stabilité suffisante aux vaccins vivants atténués, ce qui rend les possibilités de stockage de tels vaccins limités et leur éventuelle exposition lors de leur stockage ou lors de leur expédition à des températures élevées risquée. En effet, leur perte d'activité entraîne une vaccination inefficace et donc non-protectrice en cas d'infection réelle. De plus, les compositions connues sont d'une efficacité encore plus limitée lorsqu'il s'agit de stabiliser les différentes valences d'un vaccin multivalent contenant différents virus. Le vaccin tétravalent MMRV (rougeole-oreillons-rubéole-varicelle) constitue ainsi un exemple de vaccin multivalent pour lequel il n'existe actuellement pas de stabilisant en adéquation avec les quatre valences.

La présente invention a pour objet un procédé de préparation d'un vaccin contenant un virus de la varicelle vivant atténué, selon lequel (i) on cultive le virus de la varicelle vivant atténué ; (ii) on ajoute à la préparation obtenue en (i), un agent stabilisant tel que défini ci-dessous, (iii) on récolte le virus ; et (iv) on lyophilise la préparation obtenue en (iii).

Les agents stabilisants utiles dans le cadre de la présente invention ne présentent pas les inconvénients exposés ci-dessus. Ces agents stabilisants pour compositions et notamment vaccins contenant un ou plusieurs virus vivants atténués, comprennent un ou plusieurs composants choisis dans chacun des groupes que sont l'urée et ses dérivés, les acides-aminés, les disaccharides, les polyalcools, et les solutions tampons, étant entendu qu'un composant choisi dans le groupe des disaccharides est le saccharose. Dans l'agent stabilisant utile dans le cadre de la présente invention, le ou les éléments choisi(s) dans le groupe des disaccharides peut ou peuvent être présent(s) en proportion allant de 2 à 6 %. Avantageusement, le ou les éléments choisi(s) dans le groupe des disaccharides peut ou peuvent être présent(s) en proportion de 2,5 %.

Le groupe des polyalcools qui est un composant de l'agent stabilisant selon l'invention peut notamment comprendre le sorbitol et le mannitol. L'agent stabilisant selon l'invention peut comprendre le sorbitol et le mannitol. Dans le cas où l'agent stabilisant selon l'invention comprend le sorbitol, celui-ci est préférentiellement présent dans une proportion allant de 2 à 6 %. Dans le cas où l'agent stabilisant selon l'invention comprend le sorbitol, celui-ci sera avantageusement présent dans une proportion allant de 3 à 5 %. De manière particulièrement avantageuse, le sorbitol pourra être présent en proportion de 5 %. Dans le cas où l'agent stabilisant selon l'invention comprend le mannitol, celui-ci sera préférentiellement présent dans une proportion allant de 1 à 2,5 %. Dans le cas où l'agent stabilisant selon l'invention comprend le mannitol, celui-ci sera avantageusement présent en proportion de 1,5 %.

Le groupe des solutions tampons qui est un composant de l'agent stabilisant selon l'invention peut notamment comprendre les tampons phosphates et l'acide éthylène diamine tétraacétique (EDTA). De manière avantageuse, l'agent stabilisant selon l'invention comprendra un tampon phosphate. L'agent stabilisant selon l'invention pourra comprendre en outre du Dextran. Dans le cas où l'agent stabilisant selon l'invention comprend du Dextran, celui-ci sera préférentiellement présent dans une proportion allant de 1 à 4 %. Dans le cas où l'agent stabilisant selon l'invention comprend du Dextran, celui-ci sera avantageusement présent en proportion de 3 %.

L'agent stabilisant utile dans le cadre de la présente l'invention comprend de l'urée, un des dérivés de l'urée ou un mélange de ceux-ci. Des exemples de dérivés de l'urée utilisables sont l'allyurée, l'acétamide, le méthylcarbamate ou butylcarbamate. L'urée, un des dérivés de l'urée ou un mélange de ceux-ci, seront préférentiellement présents dans une proportion allant de 0,125 à 2 % ; avantageusement présent(s) en proportion de 0,5 %.

Le pH des compositions selon l'invention sera préférentiellement ajusté en fonction du ou des virions à stabiliser. L'ajustement du pH à 7 est notamment avantageux.

De nombreuses compositions et notamment les vaccins monovalents contenant essentiellement une espèce ou souche virale, ou multivalents contenant plusieurs espèces ou souches virales peuvent être stabilisés avec les agents stabilisants selon l'invention. L'objet de l'invention réside aussi dans les compositions et notamment les vaccins, stabilisés à l'aide des agents stabilisants susmentionnés.

De tels vaccins stabilisés selon l'invention peuvent par exemple comprendre au moins un virus de la famille *Herpesviridae.* La famille *Herpesviridae* comprend notamment le virus de la varicelle, le cytomégalovirus et le virus herpès simplex. Le virus de la varicelle est particulièrement fragile et thermolabile lorsqu'on le compare à d'autres virus. Les agents stabilisants selon l'invention présentent donc tout leur intérêt lorsqu'il s'agit de stabiliser un virus aussi instable que celui de la varicelle.

Les agents stabilisants selon l'invention sont applicables à d'autres virus, qui peuvent notamment être choisis parmi les *Paramyxoviridae (morbillivirus* dont la rougeole, le virus des oreillons, les virus *parinfluenza* de type 1, 2, 3, 4, les *pneumovirus*), les *Togaviridae* (rubéole), et les virus *influenza* (A, B, C).

Des mélanges obtenus à partir des virus précités ou d'autres virus et constituant des vaccins multivalents peuvent avantageusement être stabilisés à l'aide des agents stabilisants selon l'invention. On peut citer parmi les vaccins multivalents envisageables le vaccin Rougeole-Oreillons-Rubéole. Dans le contexte des associations de valences, les agents stabilisants selon l'invention présentent en plus des avantages cités plus haut des propriétés intéressantes en ce que leur aptitude à stabiliser chacune des valences permet leur association. En effet, la combinaison d'agents infectieux de différentes maladies représente un progrès économique et pratique dans la mesure où une prophylaxie permet de protéger contre plusieurs maladies contrairement aux vaccins monovalents. Cependant, la coexistence d'agents infectieux différents pose d'importants problèmes de compatibilité entre ces agents qui rend difficile une polyvaccination simultanée efficace. Les agents stabilisants selon l'invention apportent une solution à ces problèmes. La fabrication d'un vaccin tétravalent Rougeole-Oreillons-Rubéole-Varicelle est une illustration de ces problèmes d'association vaccinale car trois des valences de l'association présentent une thermoinstabilité. Lorsqu'on utilise un agent stabilisant selon l'invention, on parvient à stabiliser et à conserver un pouvoir infectant suffisant pour chacune des quatre valences.

De préférence, ces vaccins seront lyophilisés lorsque cela sera possible en vue d'améliorer encore la stabilité du vaccin final. Lors de la préparation de vaccins vivants lyophilisés lesdits vaccins seront de préférence mis en contact avec une quantité appropriée d'agent stabilisant selon l'invention. La mise en contact des virus avec l'agent stabilisant sera effectuée avant la récolte desdits virus.

L'invention sera décrite de manière plus détaillée dans la suite de la description. A titre d'illustrations, nous avons choisi d'exposer en tant qu'exemples de réalisation de l'invention la préparation d'un vaccin monovalent varicelle stabilisé ainsi que celle d'un vaccin multivalent Rougeole-Oreillons-Rubéole-Varicelle stabilisé car les problèmes de stabilité et d'association y sont particulièrement affirmés.

### EXEMPLES

### Exemple 1 : préparation d'un vaccin varicelle stabilisé selon l'invention

La souche de varicelle attenuée utilisée pour la préparation d'un vaccin stabilisé selon l'invention est la souche OKA.

On prépare un stabilisant en utilisant les composants suivants qui sont mélangés dans de l'eau p.p.i (préparation pour injection) dans les rapports qui suivent :
- Sorbitol 5 %,
- Mannitol 2,5 %,
- Saccharose 5 %,
- Dextran 3 %,
- mélange d'acides aminés,
- Urée 0.5 %,
- EDTA 0,05 %,
- Glutamate de sodium 0,05 %.

La récolte ou libération des virus des cellules infectées est réalisée soit par désintégration cellulaire (sonication, cisaillement, hautes-pressions...) dans le stabilisant, soit spontanément dans le stabilisant. On obtient ainsi après clarification une suspension virale stabilisée.

Les essais sont répartis après ajustement du titre par dilution en petits flacons puis lyophilisés suivant un cycle adapté.

### Tests de stabilité du vaccin obtenu.

Les préparations lyophilisées à tester sont réhydratées et titrées. L'activité est déterminée par le nombre d'unités formant plages (pfu) à l'aide de la numération des effets cytopathogènes obtenus en boite de 25 cm2 pour differentes dilutions des essais.

Le stabilisant-contrôle utilisé est un stabilisant traditionnel de type connu et correspondant pour exemple à celui décrit dans le brevet US 4.147.772.

| **Stabilité comparée aux différentes phases de la préparation du vaccin.** | | |
|---|---|---|
| Conditions et phases de test | Titre (pfu/ml) | |
| | Contrôle (ex.: SPGA) | Stabilisant selon l'invention |
| Pendant la récolte | x | x + 1 log |
| Après lyophilisation | x | x + 0,2 log |
| Stockage 7 jours à 37° | x | x + 1,1 log |

Dans le tableau ci-dessus, x est le titre standard.

| **Stabilité obtenue avec le stabilisant selon l'invention dans différentes conditions de stockage.** | | | | | |
|---|---|---|---|---|---|
| Durée du stockage | Perte de titre à 22.5°C (Δpfu/dose) | Durée du stockage | Perte de titre à 5°C (Δpfu/dose) | Durée du stockage | Perte de titre à -70°C (Δpfu/dose) |
| 1 mois | 0,3 | 6 mois | 0 | 9 mois | 0,1 |
| 1.5 mois | 0,3 | 12 mois | 0,1 | 18 mois | 0,3 |
| 2 mois | 0,46 | 18 mois | 0,15 | 30 mois | 0,1 |
| 3 mois | 0,54 | 24 mois | 0,28 | 36 mois | 0,06 |
| | | 30 mois | 0,35 | | |
| | | 36 mois | 0,4 | | |

Les valeurs indiquées dans le tableau ci-dessus ont été obtenues en réalisant une moyenne sur trois essais.
Le stabilisant selon l'invention permet de maintenir le titre viral pendant les différentes phases de la préparation du vaccin ainsi que pendant son stockage, qui est ici testé à quatre températures différentes.

### Exemple 2 : préparation d'un vaccin multivalent Rougeole-Oreillons-Rubéole-Varicelle stabilisé selon l'invention

Les produits viraux concentrés rougeole, oreillons, rubéole et varicelle, conservés congelés à basse température sont décongelés puis mélangés extemporanément. Ce mélange est ensuite réparti entre les différents essais additionné de produit stabilisant selon l'invention exemplifié dans l'exemple 1 précédent. On procède ainsi de façon à obtenir les proportions de solutions finales suivantes : rougeole : 1,5 volume ; oreillons : 0,01 volume ; rubéole : 1,0 volume et varicelle : 2,5 volumes. Ces différentes proportions, indiquées par rapport aux exemples, peuvent varier, selon le titre des produits viraux concentrés et ne sont pas limitatives.

Les essais sont répartis en petits flacons puis lyophilisés suivant un cycle standard. Les tests de stabilité sont réalisés en plaçant des flacons pendant 7 jours à 37°C ou 21 jours à 22°C ou 30 mois à + 4° C.

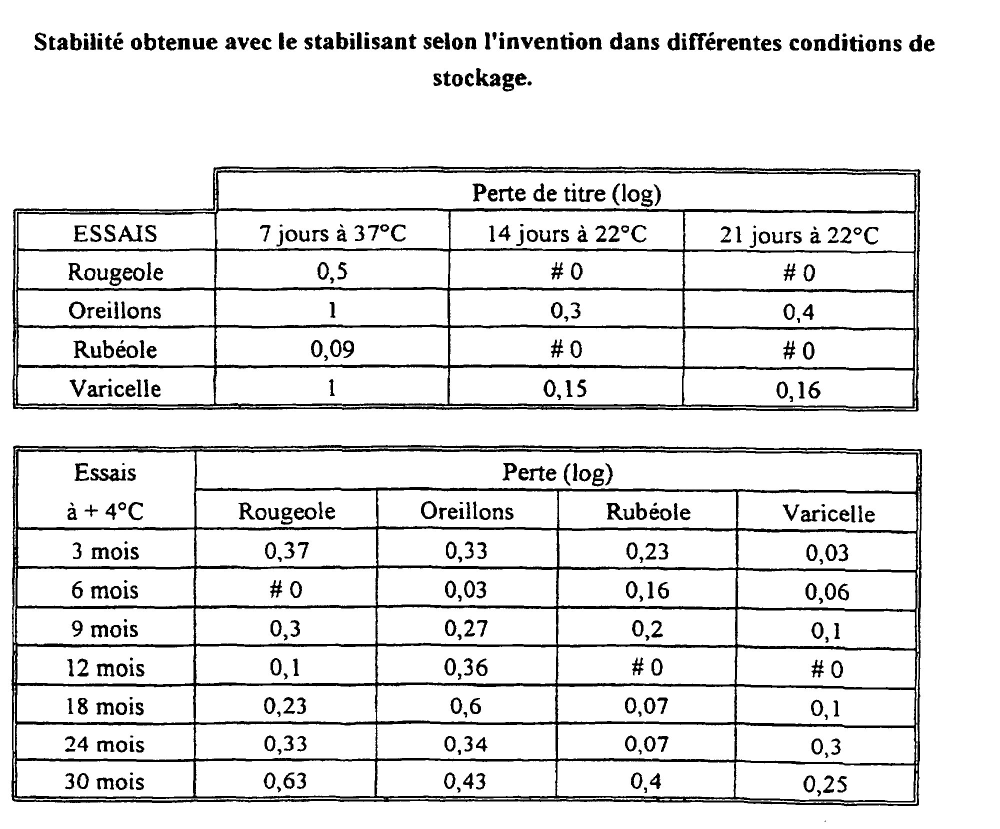

On ne constate pas de perte significativement differente de celle obtenue pour les vaccins monovalents correspondants.

## Revendications

1. Procédé de préparation d'un vaccin contenant un virus de la varicelle vivant atténué, selon lequel :
(i) on cultive le virus de la varicelle vivant atténué ;
(ii) on ajoute à la préparation obtenue en (i), un agent stabilisant pour stabiliser le virus, qui comprend :
- un ou plusieurs acide(s) aminé(s),
- du saccharose,
- un ou plusieurs polyalcool(s),
- une solution tampon, et
- de l'urée ou un dérivé de l'urée ;
(iii) on récolte le virus ; et
(iv) on lyophilise la préparation obtenue en (iii).

2. Procédé selon la revendication 1, dans lequel l'agent stabilisant comprend de 2 à 6 % de saccharose.

3. Procédé selon la revendication 2, dans lequel l'agent stabilisant comprend 5 % de saccharose.

4. Procédé selon l'une des revendications 1 à 3, dans lequel l'agent stabilisant comprend un polyalcool qui est le sorbitol.

5. Procédé selon la revendication 4, dans lequel l'agent stabilisant comprend de 2 à 6 % de sorbitol.

6. Procédé selon l'une des revendications 1 à 5, dans lequel l'agent stabilisant comprend un polyalcool qui est le mannitol.

7. Procédé selon la revendication 6, dans lequel l'agent stabilisant comprend de 1 à 2,5 % de mannitol.

8. Procédé selon l'une des revendications 1 à 7, dans lequel l'agent stabilisant comprend une solution tampon qui est un tampon phosphate.

9. Procédé selon l'une des revendications 1 à 8, dans lequel l'agent stabilisant comprend de 0,125 à 2 % d'urée ou d'un dérivé de l'urée.

10. Procédé selon l'une des revendications 1 à 9, dans lequel l'agent stabilisant comprend 0,5 % d'urée ou d'un dérivé de l'urée.

11. Procédé selon l'une des revendications 1 à 10, dans lequel l'agent stabilisant comprend en outre du dextran.

12. Procédé selon la revendication 11, dans lequel l'agent stabilisant comprend de 1 à 4 % de dextran.

13. Procédé selon la revendication 12, dans lequel l'agent stabilisant comprend 3 % de dextran.

## Claims

1. Process for preparing a vaccine which comprises an attenuated live varicella virus, according to which:
(i) the attenuated live varicella virus is cultured;
(ii) a stabilizing agent for stabilizing the virus, which agent comprises:
- one or more amino acid(s),
- sucrose,
- one or more polyalcohol(s),
- a buffer solution, and
- urea or a derivative of urea,
is added to the preparation obtained in (i);
(iii)the virus is harvested; and
(iv) the preparation obtained in (iii) is lyophilized.

2. Process according to Claim 1, according to which the stabilizing agent comprises from 2 to 6% sucrose.

3. Process according to Claim 2, in which the stabilizing agent comprises 5% sucrose.

4. Process according to one of Claims 1 to 3, in which the stabilizing agent comprises a polyalcohol which is sorbitol.

5. Process according to Claim 4, in which the stabilizing agent comprises from 2 to 6% sorbitol.

6. Process according to one of Claims 1 to 5, in which the stabilizing agent comprises a polyalcohol which is mannitol.

7. Process according to Claim 6, in which the stabilizing agent comprises from 1 to 2.5% mannitol.

8. Process according to one of Claims 1 to 7, in which the stabilizing agent comprises a buffer solution which is a phosphate buffer.

9. Process according to one of Claims 1 to 8, in which the stabilizing agent comprises from 0.125 to 2% urea or a derivative of urea.

10. Process according to one of Claims 1 to 9, in which the stabilizing agent comprises 0.5% urea or a derivative of urea.

11. Process according to one of Claims 1 to 10, in which the stabilizing agent additionally comprises dextran.

12. Process according to Claim 11, in which the stabilizing agent comprises from 1 to 4% dextran.

13. Process according to Claim 12, in which the stabilizing agent comprises 3% dextran.

## Patentansprüche

1. Verfahren zur Herstellung eines Impfstoffs, enthaltend ein abgeschwächtes lebendes Varicellavirus, gemäß dem:
(i) man das abgeschwächte lebende Varicellavirus kultiviert;
(ii) man, um das Virus zu stabilisieren, dem in (i) erhaltenen Präparat einen Stabilisator zusetzt, der umfasst:
- eine oder mehrere Aminosäure(n),
- Saccharose,
- einen oder mehrere Polyalkohol(e),
- eine Pufferlösung und
- Harnstoff oder ein Harnstoffderivat;
(iii) man das Virus erntet und
(iv) man das in (iii) erhaltene Präparat lyophilisiert.

2. Verfahren gemäß Anspruch 1, bei dem der Stabilisator 2 bis 6 % Saccharose enthält.

3. Verfahren gemäß Anspruch 2, bei dem der Stabilisator 5 % Saccharose enthält.

4. Verfahren gemäß einem der Ansprüche 1 bis 3, bei dem der Stabilisator einen Polyalkohol umfasst, der Sorbit ist.

5. Verfahren gemäß Anspruch 4, bei dem der Stabilisator 2 bis 6 % Sorbit umfasst.

6. Verfahren gemäß einem der Ansprüche 1 bis 5, bei dem der Stabilisator einen Polyalkohol umfasst, der Mannit ist.

7. Verfahren gemäß Anspruch 6, bei dem der Stabilisator 1 bis 2,5 % Mannit umfasst.

8. Verfahren gemäß einem der Ansprüche 1 bis 7, bei dem der Stabilisator eine Pufferlösung umfasst, die ein Phosphatpuffer ist.

9. Verfahren gemäß einem der Ansprüche 1 bis 8, bei dem der Stabilisator 0,125 bis 2 % an Harnstoff oder eines Harnstoffderivats umfasst.

10. Verfahren gemäß einem der Ansprüche 1 bis 9, bei dem der Stabilisator 0,5 % an Harnstoff oder eines Harnstoffderivats umfasst.

11. Verfahren gemäß einem der Ansprüche 1 bis 10, bei dem der Stabilisator außerdem Dextran umfasst.

12. Verfahren gemäß Anspruch 11, bei dem der Stabilisator 1 bis 4 % Dextran umfasst.

13. Verfahren gemäß Anspruch 12, bei dem der Stabilisator 3 % Dextran umfasst.
